# EUROPEAN PATENT SPECIFICATION

(11) **EP 0 755 671 B1**
(45) Date of publication and mention of the grant of the patent: **08.07.1998**
(21) Application number: 95914561.6
(22) Date of filing: 11.04.1995
(51) Int. Cl.: A61K 7/48, A61K 7/00

(54) **DERMATOLOGIC PREPARATION**
DERMATOLOGISCHES PRÄPARAT
PREPARATION DERMATOLOGIQUE

(30) Priority: 12.04.1994 JP 72991/94
(43) Date of publication of application: 29.01.1997
(73) Proprietor: Kao Corporation, Chuo-Ku Tokyo 103 (JP)
(72) Inventor: YAMAGUCHI, Masakazu, Tochigi 321-34 (JP); KAWAMATA, Akira, Wakayama 641 (JP); OHSU, Hiroyuki, Tochigi 321-34 (JP); TAKEMA, Yoshinori, Tochigi 307-02 (JP); IMOKAWA, Genji, Tochigi 320 (JP)
(74) Representative: Kindler, Matthias, Dr. Dipl.-Chem.
(86) International application number: JP9500710
(87) International publication number: WO9527474

(56) References cited:
- EP-A- 0 119 516
- EP-A- 0 524 108
- WO-A-93/05763
- WO-A-93/10756
- CH-A- 603 161
- US-A- 4 013 593
- US-A- 4 857 321
- US-A- 5 118 707
- US-A- 5 514 709

## Description

### TECHNICAL FIELD

The present invention relates to an external skin-care preparation, and more particularly to an external skin-care preparation which has an excellent effect of preventing the formation of wrinkles.

### BACKGROUND ART

In recent years, it has been a matter of significant concern to maintain a healthy and beautiful skin irrespective of age or sex. However, the skin is delicately affected by temperature, humidity, ultraviolet rays, cosmetic compositions, aging, diseases, stress, eating habits and the like. Therefore, various troubles such as the decrement of various functions (functions of preventing the loss of water and the like from the vital body to control the homeostatic maintenance of the body heat, protecting the body from physical and chemical stimulation and various bacteria and keeping the resilience of the skin to determine its surface form, and the like) of the skin and aging of the skin occur.

Of these, wrinkles, which are one of dermal troubles, occur due to aging or dermal aging by sunlight. More specifically, cells for producing the fibrous tissue of the dermis are made small and lessened by exposure to sunlight or with the increase in age, and particularly, collagen fibers are lost to a great extent, and so the skin is aged by degeneration of the dermis, reduction of subcutaneous adipose tissue and the like, which forms the cause of wrinkles, relaxation and loss of resilience.

Various compositions and methods have heretofore been proposed for preventing and treating or removing wrinkles caused by such an aging effect (Japanese Patent Application Laid-Open Nos. 502546/1987 and 288822/1990, etc.). Further, it has been known that the formation of wrinkles is accelerated by exposure to ultraviolet rays, UV-B. For the protection of the skin against this, it has been proposed to use an antioxidant such as α-tocopheryl acetate.

However, the effects of these conventional agents for preventing the formation of wrinkles have been insufficient.

It is therefore an object of the present invention to provide an external skin-care preparation which can sufficiently prevent the skin from loosening or wrinkling.

### DISCLOSURE OF THE INVENTION

In view of the foregoing circumstances, the present inventors have carried out an extensive investigation. As a result, it has been found that furan derivatives, benzofuran derivatives and isobenzofuran derivatives having a tertiary amino group in their molecules have marked effects of preventing the skin from loosening or wrinkling, thus leading to completion of the present invention.

According to the present invention, there is thus provided an external skin-care preparation comprising, as an active ingredient, a furan derivative represented by the following formula (1): 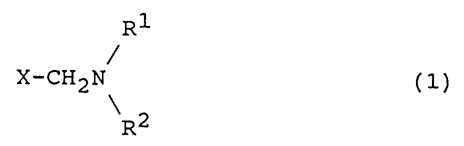 wherein X is a furan, benzofuran or isobenzofuran residue which may have a substituent group, and R¹ and R² are individually a linear, branched or cyclic alkyl group having 1-8 carbon atoms, which may be substituted by an X-CH₂-N(R³)- group (in which R³ is a linear or branched alkyl group having 1-6 carbon atoms, and X has the same meaning as defined above), or R¹ and R² may form a heterocyclic ring, which may be substituted by X-CH₂- (in which X has the same meaning as defined above), together with the adjacent nitrogen atom.

According to the present invention, there is also provided a method of preventing the formation of wrinkles, which comprises applying a composition containing the furan derivative represented by the formula (1) to the skin.

According to the present invention, there is further provided use of the furan derivative represented by the formula (1) for an agent for preventing the formation of wrinkles.

### BEST MODE FOR CARRYING OUT THE INVENTION

Examples of the furan, benzofuran or isobenzofuran residue represented by X in the formula (1) include furan-2-yl, furan-3-yl, benzofuran-2-yl, benzofuran-3-yl, isobenzofuran-2-yl and isobenzofuran-3-yl. Of these, furan-2-yl and furan-3-yl are particularly preferred. Examples of groups by which these furan, benzofuran and isobenzofuran residues may be substituted include linear or branched alkyl or hydroxyalkyl groups having 1-6 carbon atoms. Examples of the linear or branched alkyl groups having 1-6 carbon atoms include methyl, ethyl, propyl, isopropyl, butyl, isobutyl and hexyl. However, methyl and ethyl are particularly preferred in the present invention. Examples of the linear or branched hydroxyalkyl groups having 1-6 carbon atoms include hydroxymethyl, 2-hydroxyethyl, 3-hydroxypropyl and 4-hydroxybutyl. However, hydroxymethyl and hydroxyethyl are particularly preferred in the present invention.

As a more preferable group of X, may be mentioned a group represented by the following formula (2): 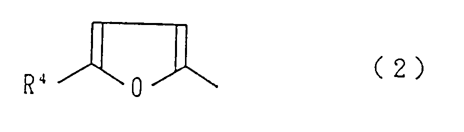 wherein R⁴ is a hydrogen atom or a linear or branched alkyl group having 1-6 carbon atoms, which may be substituted by a hydroxyl group.

Examples of the linear or branched alkyl groups represented by R¹ and R² and having 1-8 carbon atoms include methyl, ethyl, n-propyl, isopropyl and n-octyl. Examples of the cyclic alkyl groups include cycloalkyl groups having 3-6 carbon atoms, such as cyclopentyl and cyclohexyl. Of these, methyl, ethyl, n-propyl and isopropyl are particularly preferred.

Examples of the X-CH₂-N(R³)- group by which these alkyl groups may be substituted include groups in which -CH₂-N(R³)- is bonded to the furan, benzofuran or isobenzofuran residue which is represented by X and may have a substituent group. Examples of the linear or branched alkyl groups represented by R³ and having 1-6 carbon atoms include those mentioned as the examples of R¹ and R². Preferable examples of the X-CH₂-N(R³)- group include N-methyl-N-(2-furfuryl)amino, N-methyl-N-(5-methyl-2-furfuryl)amino, N-ethyl-N-(2-furfuryl)amino, N-ethyl-N-(5-methyl-2-furfuryl)amino, N-methyl-N-(5-hydroxymethyl-2-furfuryl)amino and N-ethyl-N-(5-hydroxymethyl-2-furfuryl)amino groups.

Examples of the heterocyclic ring formed by R¹, R² and the nitrogen atom include 5- or 6-membered, saturated heterocyclic rings such as pyrrolidine and piperazine rings. Examples of X of the X-CH₂- group by which such a heterocyclic ring may be substituted include the same groups as mentioned above. Preferable examples of the X-CH₂- group include furfuryl, 5-methylfurfuryl, 5-ethylfurfuryl and 5-hydroxymethylfurfuryl groups.

In the present invention, particularly preferred as the furan derivatives represented by the formula (1) are those represented by the following formula (3): 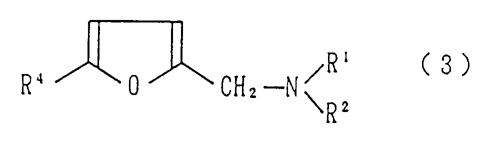 wherein R¹ and R² are individually a linear, branched or cyclic alkyl group having 1-8 carbon atoms, which may be substituted by 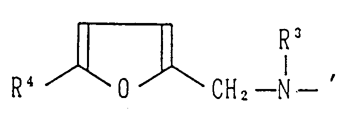 or R¹ and R² may form a pyrrolidine or piperazine ring, which may be substituted by 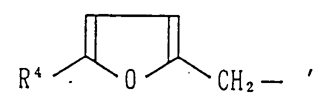 together with the adjacent nitrogen atom, and R⁴ has the same meaning as defined above.

In the above formula (3), specific examples of R⁴ include those exemplified above as the substituent groups for X.

The furan derivative represented by the formula (1), which is useful in the practice of the present invention, can be synthesized, for example, in accordance with the following Process 1 or 2: 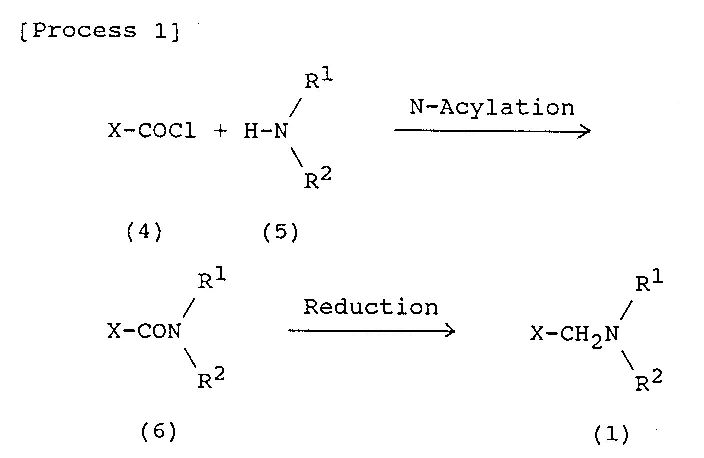 wherein R¹, R² and X have the same meaning as defined above.

More specifically, a carboxylic acid chloride (4) is reacted with an amine (5) to obtain an amide compound (6). Thereafter, the amide compound is reduced into a tertiary amine, whereby the furan derivative of the formula (1) can be synthesized.

The reaction of the carboxylic acid chloride (4) with the amine (5) is preferably conducted in accordance with the conventional N-acylation reaction, for example, in the presence of a base such as sodium hydroxide in an organic solvent which does not affect the N-acylation reaction. The reduction of the resultant amide compound (6) is preferably conducted by a method using a reducing agent such as lithium aluminum hydride or lithium boron hydride. 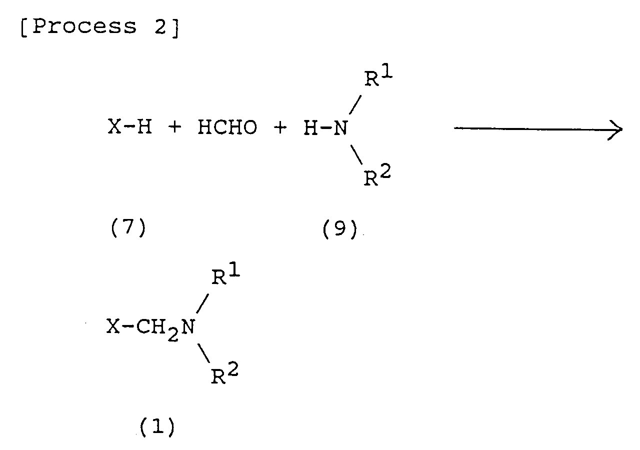 wherein R¹, R² and X have the same meaning as defined above.

More specifically, the furan derivative of the formula (1) can be synthesized by so-called Mannich reaction in which formaldehyde and an amine (5) are reacted to a furan compound (7).

This reaction is preferably conducted in the presence of an acid such as, for example, acetic acid.

The substituent group on the furan, benzofuran or isobenzofuran ring represented by X may also be introduced by alkylation or the like after completion of the reaction of Process 1 or 2.

In the external skin-care preparation according to the present invention, these furan derivatives may be incorporated either singly or in any combination thereof. No particular limitation is imposed on the amount of the furan derivative (1) to be incorporated into the external skin-care preparation. However, the amount may preferably be 0.0001-20 wt.% (hereinafter indicated merely by "%"), particularly preferably 0.001-10% based on the total weight of the external skin-care preparation.

The external skin-care preparation may be formulated in various forms by the conventional methods according to intended applications such as medicinal external skin-care preparations and cosmetic compositions.

As examples of the medicinal external skin-care preparations, may be mentioned various ointments containing one or more medicinally-effective ingredients. The ointments may include both those containing an oily base as a base and those containing an oil/water or water/oil emulsion-type base as a base.

No particular limitation is imposed on the oily base. For example, vegetable oils, animal oils, synthetic oils, fatty acids, natural and synthetic glycerides, etc. may be mentioned. No particular limitation is also imposed on the medicinally-effective ingredients. For example, one or more of analgesic and antiphlogistic agents, analgesics, disinfectants, astringents, emollients, hormones, vitamins and the like may be suitably used as needed.

When the external skin-care preparation according to the present invention is used as a cosmetic composition, there may be incorporated those ingredients employed routinely as cosmetic ingredients, such as oily substances, moisturizers, beautifiers, ultraviolet absorbents, alcohols, chelating agents, pH adjustors, antiseptics, thickeners, coloring matter and perfume bases in combination as needed.

As cosmetics, cosmetic compositions of various applications and forms may be formulated. They are used, for example, as oil/water or water/oil type emulsified cosmetics, creams, cosmetic emulsions, toilet waters, oily cosmetics, pack compositions, foundations, etc.

In order to prevent the formation of wrinkles in the present invention, it is only necessary to apply the composition containing the furan derivative to the skin of a face or the like.

### EXAMPLES

The present invention will hereinafter be described in more detail by the following Examples. However, the present invention is not limited to these examples. Synthesis Examples of the furan derivatives useful in the practice of the present invention are described prior to the description of Examples.

### Synthesis Example 1:

In a 2,000-ml three-necked flask equipped with a stirrer, reflux condenser and 500-ml dropping funnel, 90.5 g of sodium hydroxide as powder and 154 g of diethylamine were added to 1,000 ml of chloroform, and the mixture was chilled with ice water. To this mixture, a solution of 261 g of 2-furoyl chloride in 200 ml of chloroform was added dropwise over about 30 minutes. After completion of the drop addition, the mixture was continuously stirred for 1 hour and further heated under reflux for about 30 minutes. After the reaction mixture was allowed to cool, it was washed with water, and the solvent was distilled off. The resultant oily substance was distilled under reduced pressure to obtain 314 g of an amide as an intermediate.

Another three-necked flask similar to the above flask was charged with 50.5 g of lithium aluminum hydride to disperse it in 1,000 ml of dry tetrahydrofuran while chilling with ice water. To the dispersion, a solution of 211 g of the amide intermediate in 100 ml of dry tetrahydrofuran was added dropwise over 1 hour. After stirring overnight, the reaction mixture was poured little by little into 2,000 ml of ice water. The resultant slurry was filtered to remove solids. After the filtrate was separated into layers, a water layer was reextracted with 200 ml of ether, and the resultant ether extract and an organic layer were put together. After the solvent was distilled out of this solution, the resultant yellow oily substance was distilled under reduced pressure (boiling point: 69-71°C/15 mmHg) to obtain 156 g (two-stage yield: 76%) of a purified product.

The purified product thus obtained was analyzed by ¹H-NMR. As a result, it was found that the product was 2-(N,N-diethylaminomethyl)furan [Compound (1)].
¹H-NMR (CDCl₃, δ):
   7.36(dd,1H,J=0.6,1.7Hz), 6.30(dd,1H,J=1.7,3.1Hz), 6.16(dd,1H,J=0.6,3.1Hz), 3.65(s,2H), 2.52(q,4H,J=7.1Hz), 1.07(t,6H,J=7.1Hz).

### Synthesis Example 2:

A 100-ml round bottomed flask was charged with 10 ml of formalin and 20 ml of acetic acid, and the mixture was chilled with ice water in a nitrogen gas stream. To the mixture, 8.21 g of diethylamine were added while taking care that no heat was generated. The resultant mixture was continuously stirred in such a manner that the internal temperature was kept at 0-5°C. Then, 8.23 g of 2-methyl-furan were added dropwise over about 30 minutes, and the mixture was stirred overnight while keeping the internal temperature at 0-5°C. Thereafter, the reaction mixture was poured into a 10% aqueous solution of sodium hydroxide. The resulting water layer was extracted three times with 50 ml of ether. After these ether extracts and an organic layer were put together and washed with water, the solvent was distilled off. The resultant reddish brown oily substance was distilled under reduced pressure (boiling point: 88.5°C/25 mmHg) to obtain 8.47 g (yield: 52%) of a purified product.

The purified product thus obtained was analyzed by ¹H-NMR. As a result, it was found that the product was 2-methyl-5-(N,N-diethylaminomethyl)furan [Compound (2)].
¹H-NMR (CDCl₃, δ):
   6.03(m,1H), 5,87(m,1H), 3.58(s,2H), 2.53(q,4H,J=7.2Hz), 2.67(s,3H), 1.06(t,6H,J=7.2Hz).

### Synthesis Example 3:

Synthesis was conducted in accordance with the method described in Organic Syntheses Collective, Vol. III, 305 (1955) (yield: 72%). The product thus obtained was analyzed by ¹H-NMR. As a result, it was found that the product was 2-methyl-5-(N,N-dimethylaminomethyl)furan [Compound (3)].
¹H-NMR (CDCl₃, δ):
   6.05(m,1H), 5,88(m,1H), 3.38(s,2H), 2.28(s,3H), 2.45(s,6H).

### Synthesis Example 4:

Dissolved in 60 ml of dry tetrahydrofuran were 4.61 g of Compound (1) obtained in Synthesis Example 1 and 3.53 g of N,N,N',N'-tetramethylethylenediamine, and the solution was chilled to -20°C in a nitrogen gas stream. To this solution, 20 ml of n-butyllithium (1.6M hexane solution) were added, and the mixture was continuously stirred for 2 hours at room temperature. After 5.10 g of n-hexyl bromide were added, the stirring was further continued overnight. After 10 g of water were added to stop the reaction, and the solvent was distilled off, the residue was extracted with water and ether. After an organic layer was washed with water, the solvent was distilled off. The resultant reddish brown oily substance was distilled under reduced pressure (boiling point: 73-76°C/0.006 mmHg) to obtain 4.03 g (yield: 57%) of a purified product.

The purified product thus obtained was analyzed by ¹H-NMR. As a result, it was found that the product was 2-n-hexyl-5-(N,N-diethylaminomethyl)furan [Compound (4)].
¹H-NMR (CDCl₃, δ):
   6.04(d,1H,J=3.0Hz), 5.87(d,1H,J=3.01Hz), 3.61(s,2H), 2.54-2.58(m,2H), 2.50(q,4H,J=7.2Hz), 1.57-1.65(m,2H), 0.84-0.92(m,3H), 1.07(t,6H,J=7.2Hz).

### Example 1:

Compounds (1)-(4) obtained in Synthesis Examples 1-4, Compounds (5)-(11) obtained in the same manner as in Synthesis Example 1, Compounds (12)-(15) obtained in the same manner as in Synthesis Example 2 and Compounds (16) [N-(5-methylfurfuryl)dimethylamine (product of Lancaster Synthesis Ltd., Catalog No. 1741)] shown in Table 1 were used to conduct the following two tests. The results are shown in Table 2.

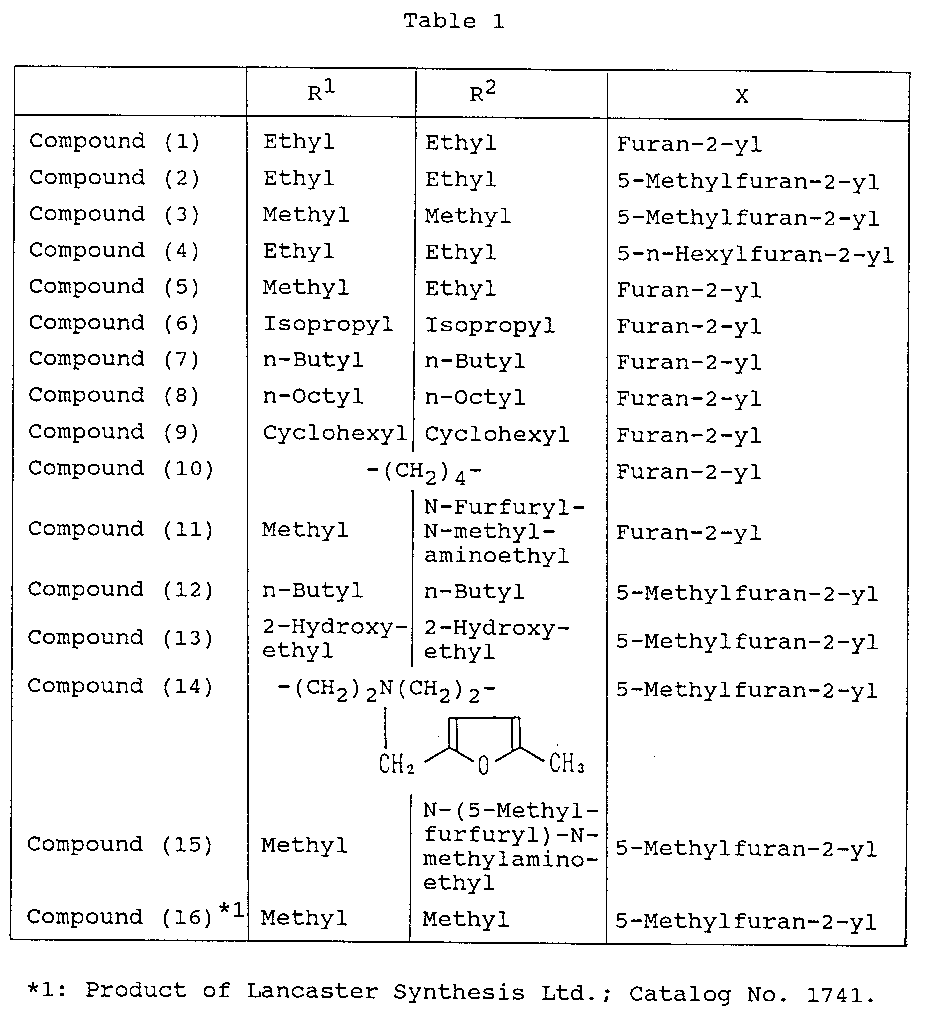

### [Test 1] Test for preventing the formation of wrinkles with hairless mice:

Each 80 µm of 5% ethanol solutions of Compounds (1)-(16) and α-tocopheryl acetate were separately applied to the backs of hairless mice (HR/ICR, aged 6 weeks at the beginning of the experiment). After about 10 minutes, the mice were exposed to UV-B rays while controlling the dose per exposure to 1 MED or less by using 6 healthy lamps (20SE, manufactured by Toshiba Corporation). The exposure was effected 5 times a week over 16 weeks. The amount of dosed energy was measured by means of a UV-Radiometer (UVR-305/365D, manufactured by TOKYO OPTICAL K.K.), whereby the total dose was determined to be 100 mJ/cm² in an amount of energy of 0.28 mW/cm² so as to give a dose of 1 MED or less per exposure. As a control, ethanol alone was applied to test in the same manner as in the samples.

After completion of the test, the degree of wrinkles formed was visually observed to evaluate the samples in accordance with the following standard (wrinkle index). Wrinkle index:
1: No wrinkle was formed;
2: Wrinkles were scarcely formed;
3: Wrinkles were somewhat formed;
4: Wrinkles were formed to a great extent.

### [Test 2] Analysis of wrinkles:

In order to analyze the particulars of the wrinkles formed in Test 1, skin replicas of the size of 1 cm² in diameter were gathered from 3 portions of the back in each of the mice using a Hydrophilic Exaflex hydrophilic vinylsilicone impression material. Each of these replicas was held horizontally and illuminated at an angle of 30 degrees from the horizontal direction, thereby finding a proportion of shadows produced by the wrinkles as an area percent by means of an image analyzer.

**Table 2**

| Compound | Wrinkle index | Area percent by image analysis (%) |
|---|---|---|
| Compound (1) | 2.11 ± 0.05 | 3.07 ± 0.24 |
| Compound (2) | 2.47 ± 0.11 | 3.47 ± 0.25 |
| Compound (3) | 2.33 ± 0.18 | 3.40 ± 0.21 |
| Compound (4) | 2.29 ± 0.28 | 3.45 ± 0.24 |
| Compound (5) | 2.23 ± 0.27 | 3.16 ± 0.23 |
| Compound (6) | 2.20 ± 0.25 | 3.27 ± 0.28 |
| Compound (7) | 2.45 ± 0.07 | 3.37 ± 0.25 |
| Compound (8) | 2.34 ± 0.18 | 3.49 ± 0.29 |
| Compound (9) | 2.49 ± 0.10 | 3.46 ± 0.31 |
| Compound (10) | 2.28 ± 0.26 | 3.37 ± 0.28 |
| Compound (11) | 2.24 ± 0.31 | 3.32 ± 0.54 |
| Compound (12) | 2.47 ± 0.28 | 2.45 ± 0.29 |
| Compound (13) | 2.34 ± 0.18 | 3.47 ± 0.25 |
| Compound (14) | 2.37 ± 0.32 | 3.31 ± 0.45 |
| Compound (15) | 2.34 ± 0.16 | 3.49 ± 0.27 |
| Compound (16) | 2.26 ± 0.19 | 3.25 ± 0.29 |
| α-Tocopheryl acetate | 3.28 ± 0.23 | 4.67 ± 0.48 |
| Control | 3.78 ± 0.08 | 6.46 ± 0.68 |

It is understood from the results shown in Table 2 that the ability of Compounds (1)-(16) to prevent the formation of wrinkles is excellent compared with α-tocopheryl acetate which is a comparative compound.

### Example 2:

The following components (1)-(7) were heated to 80°C to melt them, and the following components (8)-(11) were added to the melt. The resultant mixture was intimately mixed to prepare a W/O type cream.

| (Components) | (%) |
|---|---|
| (1) Compound (1) | 0.01 |
| (2) Cholesterol | 0.5 |
| (3) Cholesteryl isostearate | 1.0 |
| (4) Polyether-modified silicone | 1.5 |
| (5) Cyclic silicone | 20.0 |
| (6) Methylphenylpolysiloxane | 2.0 |
| (7) Methylpolysiloxane | 2.0 |
| (8) Magnesium sulfate | 0.5 |
| (9) 55% Ethanol | 5.0 |
| (10) Carboxymethylchitin (Chitin Liquid HV, product of Ichimaru Pharcos Co., Ltd.) | 0.5 |
| (11) Purified water | Balance. |

### Example 3:

The following components (1)-(10) were heated to 80°C to melt them, and the following components (11)-(12) were added to the melt. The resultant mixture was intimately mixed to prepare an O/W type cream.

| (Components) | (%) |
|---|---|
| (1) Compound (10) | 0.5 |
| (2) Polyoxyethylene (10) hardened castor oil | 1.0 |
| (3) Sorbitan monostearate | 0.5 |
| (4) Sodium stearoylmethyltaurine | 0.5 |
| (5) Cetostearyl alcohol | 2.0 |
| (6) Stearic acid | 1.8 |
| (7) Cholesterol | 1.5 |
| (8) Cholesteryl isostearate | 1.0 |
| (9) Neopentyl glycol dicaprate | 8.0 |
| (10) Methylpolysiloxane | 5.0 |
| (11) Glycerol | 5.0 |
| (12) Purified water | Balance. |

### Example 4:

The following components (1)-(7) were heated to 80°C to melt them, and the following components (8)-(10) were added to the melt. The resultant mixture was intimately mixed to prepare a sunscreen cream.

| (Components) | (%) |
|---|---|
| (1) Compound (3) | 1.0 |
| (2) Silicon-coated zinc oxide | 7.0 |
| (3) 2-Ethylhexyl p-methoxycinnamate | 3.0 |
| (4) Cholesteryl isostearate | 1.0 |
| (5) Polyether-modified silicone | 2.0 |
| (6) Methylpolysiloxane | 5.0 |
| (7) Cyclic silicone | 15.0 |
| (8) Magnesium sulfate | 1.0 |
| (9) Glycerol | 5.0 |
| (10) Purified water | Balance. |

### Example 5:

The following components (1)-(8) were heated to 70°C to melt them, and then cooled, thereby preparing a pack composition.

| (Components) | (%) |
|---|---|
| (1) Compound (9) | 5.0 |
| (2) Polyvinyl alcohol | 15.0 |
| (3) Sodium carboxymethylcellulose | 5.0 |
| (4) Propylene glycol | 3.0 |
| (5) Ethanol | 8.0 |
| (6) Purified water | Balance |
| (7) Perfume base | 0.5 |
| (8) Antiseptic | q.s. |

### Example 6:

The following components (1)-(6) were heated to 80°C to melt them, and then cooled, thereby preparing an ointment.

| (Components) | (%) |
|---|---|
| (1) Compound (9) | 10.0 |
| (2) White petrolatum | Balance |
| (3) Cholesteryl isostearate | 3.0 |
| (4) Liquid paraffin | 10.0 |
| (5) Isostearyl glyceryl ether | 1.0 |
| (6) Glycerol | 10.0. |

### Example 7:

The following components (1)-(4) were mixed into Solution A. On the other hand, the following components (5)-(8) were mixed into Solution B. This Solution B was added to Solution A to dissolve Solution B in Solution A, thereby preparing a toilet water.

| (Components) | (%) |
|---|---|
| (1) Citric acid | 0.1 |
| (2) Zinc sulfocarbolate | 0.2 |
| (3) Glycerol | 5.0 |
| (4) Purified water | Balance |
| (5) Compound (5) | 1.0 |
| (6) Polyoxyethylene (20) oleyl alcohol ether | 1.0 |
| (7) Ethanol | 20.0 |
| (8) Perfume base | 0.2. |

The external skin-care preparations according to the present invention prepared in Examples 1-7 each had an excellent effect for preventing the formation of wrinkles.

### INDUSTRIAL APPLICABILITY

The external skin-care preparations according to the present invention are excellent in effect to prevent the formation of wrinkles caused by exposure to ultraviolet rays, in particular, UV-B rays and are hence useful as external skin-care preparations capable of preventing the formation of wrinkles.

## Claims

1. An external skin-care preparation comprising, as an active ingredient, a furan derivative represented by the following formula (1): 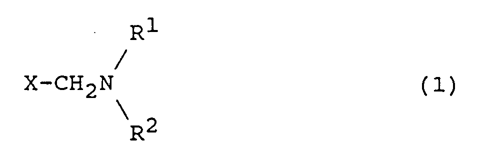 wherein X is a furan, benzofuran or isobenzofuran residue which may have a substituent group, and R¹ and R² are individually a linear, branched or cyclic alkyl group having 1-8 carbon atoms, which may be substituted by an X-CH₂-N(R³)- group (in which R³ is a linear or branched alkyl group having 1-6 carbon atoms, and X has the same meaning as defined above), or R¹ and R² may form a heterocyclic ring, which may be substituted by X-CH₂- (in which X has the same meaning as defined above), together with the adjacent nitrogen atom.

2. The external skin-care preparation according to Claim 1, wherein X in the formula (1) is represented by the following formula (2): 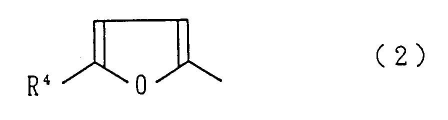 wherein R⁴ is a hydrogen atom or a linear or branched alkyl group having 1-6 carbon atoms, which may be substituted by a hydroxyl group.

3. The external skin-care preparation according to Claim 1, which is an external skin-care preparation capable of preventing the formation of wrinkles.

4. A method of preventing the formation of wrinkles, which comprises applying a composition containing the furan derivative according to Claim 1 or 2 to the skin.

5. Use of the furan derivative according to Claim 1 or 2 for an agent for preventing the formation of wrinkles.

## Patentansprüche

1. Externes Hautpflegepräparat, umfassend ein Furanderivat, dargestellt durch die folgende Formel (1), als aktiven Bestandteil: 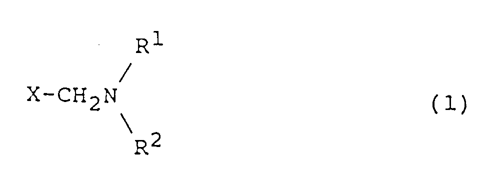 worin X ein Furan-, Benzofuran- oder Isobenzofuranrest ist, der eine Substituentengruppe haben kann, und R¹ und R² individuell eine lineare, verzweigte oder cyklische Alkylgruppe mit 1 bis 8 Kohlenstoffatomen sind, die durch eine X-CH₂-N(R³)-Gruppe substituiert sein kann (worin R³ eine lineare oder verzweigte Alkylgruppe mit 1 bis 6 Kohlenstoffatomen ist und X die gleiche Bedeutung wie oben beschrieben aufweist), oder worin R¹ und R² einen heterozyklischen Ring, der durch X-CH₂- (worin X die gleiche Bedeutung wie oben definiert aufweist), substituiert sein kann, zusammen mit dem benachbarten Stickstoffatom bilden können.

2. Externes Hautpflegepräparat nach Anspruch 1, worin X in der Formel (1) durch die folgende Formel (2) dargestellt ist: 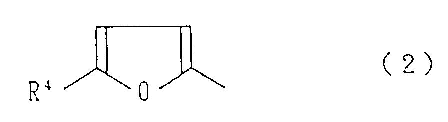 worin R⁴ ein Wasserstoffatom oder eine lineare oder verzweigte Alkylgruppe mit 1 bis 6 Kohlenstoffatomen ist, die durch eine Hydroxylgruppe substituiert sein kann.

3. Externes Hautpflegepräparat nach Anspruch 1, das ein externes Hautpflegepräparat ist, das die Faltenbildung verhindern kann.

4. Verfahren zur Verhinderung der Faltenbildung, umfassend das Auftragen einer Zusammensetzung, die das Furanderivat nach Anspruch 1 oder 2 enthält, auf die Haut.

5. Verwendung des Furanderivates nach Anspruch 1 oder 2 für ein Mittel zur Verhinderung der Faltenbildung.

## Revendications

1. Préparation pour soins externes de la peau comprenant, en tant qu'ingrédient actif, un dérivé du furane représenté par la formule suivante (1) : 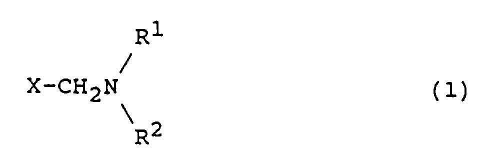 dans laquelle X est un résidu furane, benzofurane ou isobenzofurane qui peut comporter un groupement substituant, et R¹ et R² sont chacun un groupement alkyle linéaire, ramifié ou cyclique comprenant de 1 à 8 atomes de carbone, qui peut être substitué par un groupement X-CH₂-N(R³)- (dans lequel R³ est un groupement alkyle linéaire ou ramifié comportant de 1 à 6 atomes de carbone, et X a la même signification que celle définie ci-dessus), ou bien R¹ et R² peuvent former un hétérocycle, qui peut être substitué par X-CH₂- (dans lequel X a la même signification que celle définie ci-dessus), avec l'atome d'azote adjacent.

2. Préparation pour soins externes de la peau selon la revendication 1, dans laquelle X dans la formule (1) est représenté par la formule suivante (2) : 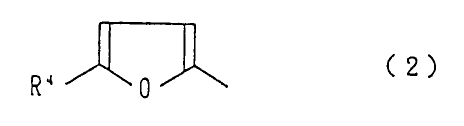 dans laquelle R⁴ est un atome d'hydrogène ou un groupement alkyle linéaire ou ramifié comportant de 1 à 6 atomes de carbones, qui peut être substitué par un groupement hydroxyle.

3. Préparation pour soins externes de la peau selon la revendication 1, qui est une préparation pour soins externes de la peau capable d'empêcher la formation de rides.

4. Procédé destiné à empêcher la formation de rides, qui comprend l'application d'une composition contenant le dérivé de furane conforme à la revendication 1 ou 2 sur la peau.

5. Utilisation du dérivé de furane selon la revendication 1 ou 2 en tant qu'agent destiné à empêcher la formation de rides.
